# EUROPEAN PATENT SPECIFICATION

(11) **EP 3 383 202 B1**
(45) Date of publication and mention of the grant of the patent: **02.04.2025**
(21) Application number: 16802067.5
(22) Date of filing: 29.11.2016
(51) Int. Cl.: A23L 33/17, A23L 33/10, A61K 38/01

(54) **HIGH PROTEIN ENTERAL TUBE FEED FOR ICU PATIENTS**
PROTEINREICHE ENTERALE SONDENERNÄHRUNG FÜR INTENSIVPATIENTEN
ALIMENTATION ENTÉRALE PAR SONDE À PROTÉINE ÉLEVÉE POUR DES PATIENTS D'UNITÉ DE SOINS INTENSIFS

(30) Priority: 30.11.2015 WO PCT/EP2015/197108
(43) Date of publication of application: 10.10.2018
(73) Proprietor: Fresenius Kabi Deutschland GmbH, 61352 Bad Homburg (DE)
(72) Inventor: BRITO DE LA FUENTE, Edmundo, 61381 Friedrichsdorf (DE); KEIM, Susanne, 61352 Bad Homburg (DE); PESTANA, Ericka, 61348 Bad Homburg (DE)
(74) Representative: Fresenius Kabi Deutschland GmbH
(86) International application number: PCT/EP2016/079074
(87) International publication number: WO 2017/093217

(56) References cited:
- US-A1- 2011 081 400
- US-A1- 2011 081 424
- US-A1- 2013 210 715
- US-A1- 2014 051 627
- PETER JM WEIJS ET AL: "Early high protein intake is associated with low mortality and energy overfeeding with high mortality in non-septic mechanically ventilated critically ill patients", CRITICAL CARE, BIOMED CENTRAL LTD., LONDON, GB, vol. 18, no. 6, 14 December 2014 (2014-12-14), pages 701, XP021207712, ISSN: 1364-8535, DOI: 10.1186/S13054-014-0701-Z
- "Product Information: Vital High Protein Vital High Protein High Protein, Low Fat Therapeutic Nutrition", 13 November 2015 (2015-11-13), XP055247629, Retrieved from the Internet <URL:http://static.abbottnutrition.com/cms-prod/abbottnutrition.com/img/Vital-High-Protein.pdf> [retrieved on 20160204]

## Description

### FIELD OF THE INVENITON

The present disclosure relates to enteral tube feeds of high protein density that are sufficiently tolerable to provide high amounts of protein and other required nutrients to ICU patients. The enteral tube feeds herein comprise a protein component, a lipid component, a carbohydrate component and, preferably, vitamins and minerals (to be nutritionally complete). The present disclosure further relates to the use of such enteral tube feeds in the treatment of ICU patients.

### BACKGROUND OF THE INVENTION

In the Intensive Care Unit (ICU), there is a general need for enteral tube feeds providing a high amount of protein with a relatively small volume of formula as well as with a comparably low amount of calories, in particular from lipids.

Such enteral tube feeds should be particularly well tolerated by the stressed digestive system of ICU patients. As this condition is not sufficiently fulfilled by state of the art tube feeds, it is often recommended to use trophic feeding ("underfeed") patients for the first hours / days after admission to the ICU. Also, even though there is accumulating evidence, that in general enteral feeding is superior to parenteral feeding in terms of clinical outcome, often parenteral nutrition instead of enteral nutrition is provided in order to avoid digestibility issues or aspiration.

Reoccurring issues with nutritionally complete high protein enteral tube feeds are lack of stability and high viscosities of the resulting emulsions. This behaviour is particularly pronounced in the presence of minerals which are required to provide a nutritionally complete formula. A solution may be to provide a set of enteral modules in order to separately provide protein and minerals.

However, in the ICU, patient handling and monitoring is complex enough already without having a patient specific nutrient protocol requiring application of different tube feed modules. Thus a complex nutritional system is likely to result in low compliance, in particular in view of the fact that no feeding or trophic feeding is an accepted practice in particular in the first hours / days of the ICU stay.

Moreover, for being suitable to be safely administered to an ICU patient, enteral tube feeds need to be sufficiently sterile. Thus, it is desirable that they are stable against heat sterilization, preferably repeated heat sterilization.

Vital High Protein by Abbott Laboratories represents a peptide-based therapeutic nutrition to manage inflammation and symptoms of GI intolerance during low-fat, high protein diet. US2014051627A discloses a composition and method for providing nutritional support to obese patients. The composition has an energy density between 0.4 and 0.9 kcal/ml and comprises a protein source which comprises at least 30 percent by weight whey protein and provides at least 30 percent of the total calories of the composition.

There is a need for enteral tube feeds providing a high protein density and being sufficiently tolerable to the stressed digestive system of the ICU patient. In particular there is a need for such a composition being additionally adaptable towards being nutritionally complete. Moreover, such enteral tube feeds should provide high amounts of protein with a low volume of formula and low amounts of calories.

### SUMMARY OF THE INVENTION

The inventors found that, despite e.g. their high protein density, the compositions of the present disclosure are stable emulsions that can be repeatedly sterilized and are well tolerated by ICU patients.

These properties together enable the initiation of enteral feeding as early as possible after admission to the ICU in order to reach a target protein intake faster than with conventional enteral tube feeds (e.g. having a protein density of about 15-25EN%).

This high tolerability is believed to be related to the use of a defined protein hydrolysate in combination with low Gl carbohydrates and a lipid component that preferably is high in MCT. Tolerability may further be improved by relatively low amounts of energy delivered by the lipid component.

Accordingly, in a first aspect the present invention relates to an enteral tube feed as defined in claims 1-4 for use in prevention and treatment of protein malnutrition in ICU patients.

In a second aspect the present invention relates to an enteral tube feed comprising a lipid component, a carbohydrate component and at least 30 EN % of a protein component based on the total energy content of the enteral tube feed, wherein the protein component comprises a peptide fraction consisting of di-, tri- and oligopeptides with a molecular weight of at most 1kD, wherein the peptide fraction provides at least 10 EN%, preferably at least 15 EN %, more preferably at least 20 EN % of the total energy content of the enteral tube feed and wherein the carbohydrate component consists of at least 50 wt% isomaltulose, wherein the enteral tube feed comprises 10-12 wt% protein based on the total weight of the enteral tube feed and wherein the enteral tube feed has an energy density of 1.1-1.4 kcal/mL.

Also disclosed herein is an enteral tube feed for ICU patients comprising at most 30 EN % of a lipid component.

Further disclosed herein is a process for making such a composition.

Further discloses herein is an enteral tube feed that is stable against repeated sterilization under heat.

Also disclosed herein is a dosage regime for administration to an ICU patient, wherein the administration is started no later than 48 hours after admission to the ICU, preferably no later than 24 hours, preferably no later than 12 hours, most preferred within the first 1-6 hours after admission to the ICU.

### DETAILED DESCRIPTION OF THE INVENTION

### Definitions

"High protein" as used herein refers to nutritional compositions wherein the protein component provides at least 30 EN%, of the total energy of the nutritional composition. Such nutritional compositions comprise 10 - 12 wt% of protein based in the total weight of the enteral tube feed. According to the present disclosure, there will typically be an upper limit to protein content such as at most 40 EN%, preferably at most 35 EN%

"Caloric density", or energy density as used herein refers to the amounts of calories provided per volume of enteral tube feed as applied to the patient / in ready to use form. The enteral tube feeds herein have a caloric density of between 1.1 - 1.4 kcal/ml, for example 1.2 kcal/mL.

"Ready-to-use" refers to the final form of the enteral tube feed as administered to a patient. Typically, the enteral tube feed herein are pre-packed in a ready to use format. I.e. sold in separately packed dose units that do not require any further dilution etc.

"Enteral tube feeding" refers to the delivery of nutrition (enteral tube feed) directly into the gut via a tube. The tube is usually placed into the stomach, duodenum or jejunum via either the nose, mouth or the direct percutaneous route.

"Enteral tube feed" herein refers to a synthetically produced tube feed composition. Enteral tube feeds as used herein typically are provided in liquid form. Thus, enteral tube feeds are artificial products produced by mixing / dissolving bulk ingredients in order to have a controllable nutrient profile whereby said ingredients are typically provided in solid form (e.g. powders) or liquid from (e.g. oils). The term "enteral tube feed" as used herein excludes traditional food that is e.g. purred and / or diluted.

"Nutritionally complete" refers to enteral tube feeds suitable as sole source of nutrition. Nutritionally complete enteral tube feeds comprise at least a protein component, a lipid component, a carbohydrate component, vitamins and minerals. The nutrient requirements with respect to minerals and vitamins, are known to the skilled artisan and can be found in standard nutritional guidelines such as EU commission directive 1999/21/EC (see Table 1 copied herein). Suitable nutrients according to the present disclosure fulfil the requirements of / are listed in regulation (EU) No 609/2013.

A composition "consisting of" a number of ingredients or components is to be understood as comprising no other than the named ingredients or components. In case ranges for amounts of ingredients or components are given, the individual amount of all ingredients or components within the composition has of course also to be adapted such that the sum of all amounts of all present ingredients or components adds up to 100wt%.

"UHT-treatment" as used herein aims at killing of microorganisms. UHT treatment may be carried out with a first homogenization step with a pressure of 200-550 bar followed by UHT at 138°C-143°C for 6-10 seconds.

"Malnutrition" as used herein refers to one or both of Option I: body mass index (BMI, kg/m2) <18.5; Option II: the combined finding of unintentional weight loss (mandatory) and at least one of either reduced BMI or a low fat free mass index (FFMI). Weight loss is defined as either >10% of habitual weight indefinite of time, or >5% over 3 months. Reduced BMI is <20 or <22 kg/m2 in subjects younger and older than 70 years, respectively. Low FFMI is <15 and <17 kg/m2 in females and males, respectively.

An ICU patient as used herein refers to a patient admitted to an intensive care unit. An intensive care unit (ICU), also known as an intensive therapy unit or intensive treatment unit (ITU) or critical care unit (CCU), is a special department of a hospital or health care facility that provides intensive care medicine. Intensive care patients are patients with severe and life-threatening illnesses and injuries, which require constant, close monitoring and support from specialist equipment and medications in order to ensure normal bodily function. ICU's are typically distinguished from normal hospital wards by a higher staff-to-patient ratio and access to advanced medical resources and equipment that is not routinely available elsewhere. Common conditions that are treated within ICUs include ARDS (acute respiratory distress syndrome), trauma, multiple organ failure and sepsis. Patients may be transferred directly to an intensive care unit from an emergency department if required, or from a ward if they rapidly deteriorate, or immediately after surgery if the surgery is very invasive and the patient is at high risk of complications. Herein, ICU patients are preferably mechanically ventilated ICU patients.

### Enteral tube feeds

The enteral tube feeds herein comprise nutrients in predetermined and controllable amounts. An enteral tube feed according to the present disclosure comprises a protein component, a lipid component, a carbohydrate component, and, preferably minerals and vitamins. Optionally, such a nutritional composition may further comprise dietary fibres and/or further ingredients known as food additives.

The enteral tube feed herein is liquid and will be provided as an oil-in-water emulsion (O/W).

The enteral tube feed will be adapted to have a high protein density of at least 30 EN% based on the total energy content of the enteral tube feed.

The enteral tube feed will have a density of between 1.1 - 1.4kcal/mL.

The enteral tube feed herein comprises a lipid component, a protein component, a carbohydrate component, wherein
a. the protein component provides at least 30 EN % of the total energy of the composition
b. the lipid component may provide at most 30 EN % of the total energy of the composition;
c. the carbohydrate component may provide at least 20 EN %, preferably at least 30 EN %, more preferred at least 40 EN %, of the total energy of the composition;

Preferably, the enteral tube feed according to the present disclosure comprises a lipid component, a protein component, a carbohydrate component, wherein
a. the protein component provides 30-40 EN%, preferably 30-35 EN%, more preferably 32-34 EN%;
b. the lipid component provides 20-30 EN%, preferably 22-25 EN%; and
c. the carbohydrate component provides 30-50 EN% preferably 35-45 EN%, more preferably 40-44 EN%.

In preferred embodiments, the amount of water comprised in the present composition represents 70-90 vol%, preferably 75-85 vol% based on the total volume of the enteral tube feed (ready to use).

The enteral tube feed will typically have an osmolarity of 500 - 700, preferably of 550 - 650 mosmol/L.

### Protein component

The protein component herein comprises hydrolysed protein.

The protein component comprises a peptide fraction consisting of di-, tri- and oligopeptides with a molecular weight of at most 1kD, wherein the peptide fraction provides at least 10 EN%, preferably at least 15 EN %, more preferably at least 20 EN % of the total energy content of the enteral composition.

Such a protein component is believed to improve tolerability, promote gastric emptying, decrease gastroesophageal reflux and thereby reduce the probability for aspiration, in particular in mechanically ventilated ICU patients. Moreover, such a protein component supports stability of the enteral tube feed and improves its viscosity, in particular in combination with minerals, thereby providing for a liquid product that can be administered by a tube with small diameter.

In preferred embodiments, the protein component comprises hydrolysed whey protein, even more preferably, the protein component consists of hydrolysed whey protein.

The enteral tube feed herein comprises 10 - 12 wt% of protein, such as e.g. 10 wt% of protein based on the total weight of the enteral tube feed.

In preferred embodiments, the protein component provides at least 30-40 EN%, preferably 30-35 EN%, more preferably 32-34 EN% based on the total energy of the enteral tube feed.

Preferably, the protein to water ratio of the present nutritional composition is between 1.0/10 [g/g] and 1.5/10 [g/g], preferably 1.2/10 [g/g] and 1.3/10 [g/g], such as e.g. 1.25/10 [g/g].

### Carbohydrate component

The carbohydrate component may comprise one or more carbohydrate sources. The carbohydrate component predominantly consists of isomaltulose. With respect to the carbohydrate component herein, "predominantly" refers to presence of at least 50wt%, preferably at least 55wt%, more preferably at least 60wt% based on the total weight of the carbohydrate component.

The carbohydrate component typically provides at least 20 EN %, preferably at least 30 EN %, more preferred at least 40 EN %, based on the total energy of the enteral tube feed.

Preferably, the carbohydrate component provides 30-50 EN% preferably 35-45 EN%, more preferably 40-44 EN% based on the total energy of the enteral tube feed.

### Fibre

The nutritional composition herein may comprise ingredients declarable as dietary fibres. Suitable dietary fibres may be included in the carbohydrate component and may be selected from the group consisting of cocoa powder, inulin, wheat dextrine, cellulose, microcrystalline cellulose, soy polysaccharides, tapioca dextrine, xanthan, fructooligosaccharides, galactooligosaccharides, at least partially hydrolysed guar gum, acacia gum, pectin, oat fibre, poly dextrose, resistant starch, hemicellulose and mixtures thereof.

A preferred fibre mixture consists of 80-99 wt%, preferably 90 - 96wt% of soluble dietary fibre and 1-20 wt%, preferably 4-10wt% of insoluble dietary fibre each based on the total weight of dietary fibre.

The fibre mixture may consist of 80-99 wt%, preferably 85 - 95 wt% of fermentable dietary fibre and 1-20 wt%, preferably 5-15 wt% of non-fermentable dietary fibre each based on the total weight of dietary fibre.

The fibre mixture may comprise or consist of tapioca dextrin and cellulose.

Preferably, the enteral tube feed comprises between 0.1 and 2.0 wt%, preferably 0.5 to 1.0 wt% of dietary fibre, such as the fibre mixture described hereinabove, based on the total weight of the enteral tube feed.

### Lipid component

The lipid component may comprise one or more lipid sources, such as lipids of animal and / or vegetable origin.

The lipid component of the enteral tube feed is specifically adapted to the needs of ICU patients. It is consciously designed to prevent / reduce lipid overfeeding and to improve tolerability. At the same time, the lipid component herein is designed to counteract inflammatory processes, which ICU patients are at particular risk of.

With its relatively low amount of lipids, the tube feed is specifically adapted to the needs of ICU patients. ICU patients are typically heavily medicated inter alia with drugs provided in lipid emulsions, such as certain sedating drugs. Typical examples are propofol lipid emulsions. Moreover, such drugs are often provided by constant infusion.

Therefore, standard medical treatment in the ICU typically requires administration of a non-negligible amount of calories resulting from drugs applied as lipid emulsions. Often the need for such drug lipid emulsions is highest within the first few days after admission to the ICU.

However, overfeeding of ICU patients, in particular lipid overfeeding, can result in serious side effects, hyperlipidaemia being just one example.

Accordingly, standard enteral feeds, that typically have an energy contribution of the lipid component of at least 30EN% and at the same time provide no more than 25EN% of protein, each based on the total energy content of said standard enteral feed, are not ideal for ICU patients, in particular when higher doses of protein should be applied as early as possible after admission to the ICU.

Therefore, while providing at least 30EN% of protein, the enteral tube feed of the present disclosure preferably comprises less less than 30EN% of lipids based on the total energy content of the enteral tube feed.

In general, for the present enteral tube feed, less than 5wt%, e.g. 2-4wt% of the total weight of the composition may be provided by the lipid component.

The lipid component comprises medium chain triglycerides (MCT), for example in form of an MCT-oil. MCT herein refers to triglycerides of fatty acids with a chain length of 6-12 carbon atoms (C6-C14), preferably C6, C8, C10, C12 and C14.

Preferably, MCT are a major lipid source. Accordingly, the lipid component may comprise at least 30wt% of MCT, preferably, at least 35wt%, for example 40wt% of MCT based on the total weight of the lipid component. The MCT may be present in amounts of 30-50wt%, 35-45wt%, such as 37-43wt% based on the total weight of the lipid component.

In terms of energy contribution, the MCT may provide 8-12EN% based on the total energy of the enteral tube feed.

In addition to MCT, the lipid component may comprise long chain fatty acids, preferably in the form of triglycerides comprising saturated (SFA), mono-unsaturated (MUFA) and polyunsaturated fatty acids (PUFA).

The SFA may provide 2-3EN% based on the total energy content of the enteral tube feed.

The MUFA may provide 4-8EN% based on the total energy content of the enteral tube feed.

The PUFA may provide 4-8EN% based on the total energy content of the enteral tube feed.

The PUFA may comprise linoleic acid, alpha linolenic acid, EPA and DHA.

In the lipid component, the ratio of n6/n3 PUFA (g/g) may be between 0.4-0.6.

For example, in addition to the MCT-oil, the lipid component may comprise an oil of marine origin, such as fish oil and oil from plant origin, such as rapeseed-oil.

The marine oil may be provided by fish oil.

The vegetable oil may be provided by rapeseed oil..

Therefore, in one embodiment the lipid component consists of a mixture of MCT oil, fish oil and rapeseed oil.

### Vitamins and Minerals

To be regarded as nutritionally complete, nutritional compositions have to comprise vitamins and minerals in addition to the protein, lipid and carbohydrate components.

Suitable vitamins to be included in the composition in order to render it nutritionally complete according to the present disclosure are Vitamin A, Vitamin D, Vitamin K, Vitamin C, Thiamin, Riboflavin, Vitamin B6, Niacin, Folic acid, Vitamin B12, Pantothenic acid, Biotin and Vitamin E. An example for rendering a nutritional composition complete in vitamins is given in table 1.

Suitable minerals to be included in the composition in order to render it nutritionally complete according to the present disclosure are Sodium, Chloride, Potassium, Calcium, Phosphorus, Magnesium, Iron, Zinc, Copper, Iodine, Selenium, Manganese, Chromium and Molybdenum. Optionally, Fluoride may be included. An example for rendering a nutritional composition complete in minerals is given in table 1.

### Additives

Nutritional compositions optionally comprise food additives. Additives are typically present in total amounts of less than 10wt%, 5wt% or even less than 1wt% based on the total weight of the nutritional composition. Exemplary additives are choline, beta-carotene, lutein, lycopene, caffeine, lecithin, taurine, carnitine, myo-inositol, colorants, aroma and mixtures thereof. Aromas may be caramel, vanilla, yoghurt, chocolate, coffee, cappuccino, fruit aromas and the like.
The additives may include stabilisers and emulsifiers. Preferably, the stabilisers are selected from microcrystalline cellulose E460 and Sodium Carboxymethylcellulose E466 (preferably used in combination) and the emulsifiers are selected from mono-diglycerides such as citric acid ester of mono-diglyceriede E472c.

### Use in treatment of ICU patients

The enteral tube feed may be used in prevention and treatment of protein malnutrition in ICU patients, preferably in ICU patients requiring drugs provided in from of lipid emulsions. For example, the enteral tube feed may be used in prevention and treatment of malnutrition and / or protein depletion in ICU patients receiving propofol in form of lipid emulsions and / or parenteral nutrition in form of lipid emulsions.

The enteral tube feed may be used for improving the clinical outcome of the ICU patients. In particular an improved clinical outcome when compared to standard enteral tube feeds. Improved clinical outcome may be reflected in a shorter ICU stay, faster start of target enteral nutrient intake, such as target protein intake, decrease in days on mechanical ventilation or even in a faster and/or better recovery from the underlying severe illness or injury having required the admission to an ICU, e.g. trauma, sepsis, stroke, infections, myocardial infarction, anaphylactic shock or intoxication. Improved clinical outcome may also be reflected by an improved (reduced) SOFA (Sequential Organ Failure Assessment) score. Preferably, improved clinical outcome is reflected by one or more of a shorter ICU stay, less days requiring mechanical ventilation and faster start of target enteral nutrient intake, such as target protein intake.

The enteral tube feed may be used to achieve a daily target protein intake of 1.0-2.0g, preferably 1.2-1.8 g, more preferably, 1.4-1.8g of protein per kg bodyweight as early as possible after admission to the ICU. The target protein intake is calculated relative to the admission body weight. Accordingly, the enteral tube feed may be used to achieve a daily target protein intake of 1.0-2g, preferably 1.2-1.8 g, more preferably, 1.4-1.8g of protein per kg bodyweight in ICU patients, preferably in ICU patients requiring drugs provided in from of lipid emulsions, e.g. in ICU patients receiving propofol in form of lipid emulsions and / or (supplemental) parenteral nutrition in form of lipid emulsions.

The enteral tube feed may be used to provide a daily dose of protein of 1.0-2g, preferably 1.2-1.8 g, more preferably, 1.4-1.8g of protein per kg bodyweight in ICU patients, at least in the acute phase (24h - 48h hours after admission to an ICU)

Administration of the enteral tube feed disclosed herein is preferably started no later than 48 hours after admission to the ICU, preferably no later than 24 hours, preferably no later than 12 hours, most preferred within the first 1-6 hours after admission to the ICU.

### Daily dose and dose unit

An average daily dose based on the needs of an ICU patient of average weight is described below.

The enteral tube feed of the present disclosure may provide an average daily dose of 1000-1400kcal.

The enteral tube feed of the present disclosure may provide an average daily dose of 80-120g of the protein component.

The enteral tube feed of the present disclosure may provide an average daily dose of 30-35 g of the lipid component.

The enteral tube feed of the present disclosure may provide an average daily dose of 110-150g of the carbohydrate component.

The enteral tube feed of the present disclosure may provide an average daily dose of 3-10g dietary fibre.

The enteral tube herein may be adapted to provide 20 - 25 kcal/kg BW/day in the acute phase (24h - 48h hours after admission to an ICU) and an increased daily dose of calories of 25 - 30 kcal/kg BW/day in the stabilized patient (>48 hours after admission to the ICU).

For ease of use and in order to increase compliance, the average daily dose may be provided in packages (dose units). Preferably, the average daily dose is provided in one or two packages (dose units).

The average daily dose may be provided in a single dose unit, for example the average daily dose may be provided in a single tube feed package, such as a tube feed package comprising 800-1200mL of the enteral tube feed disclosed herein. Preferably, the average daily dose may be provided in two dose units wherein each provides half of the average daily dose unit, e.g. for an average bodyweight of 70kg or 75kg. For example, half of the average daily dose may be provided in a single tube feed package, such as a tube feed package comprising 400-600mL of the enteral tube feed disclosed herein.

### Process

The enteral tube feed herein is obtainable by the following process.

In a first step, the protein is mixed with water, the water may optionally be premixed with other ingredients in powder form.

In a second step, ingredients in oil form are added to and mixed with the mixture obtained by the first step.

In a third step, the mixture obtained by the second step is homogenized at 200-550bar.

In a fourth step, the mixture obtained by the third step is subjected to UHT treatment, preferably at 138-143°C for 6-10 seconds. The fourth step optionally includes an aseptic homogenization at 100-300bar after the UHT treatment.

In a fifth step, the mixture obtained in the fourth step is filled into a package holding a dose unit and the package is sealed. Optionally, this step may be carried out under aseptic conditions.

In an optional sixth step, the package obtained in the fifth step may be subjected to a further sterilization at 116-121°C for 1-20 minutes.

### EXAMPLES

### Stability test / UHT treatment

Sample enteral tube feeds according to the table below where prepared and subjected to UHT treatment UHT treatment was carried out with a first homogenization step with a pressure of 200-550 bar followed by UHT at 138°C-143°C for 6 seconds.

However, for EN test 2, UHT treatment was not possible. The UHT was blocked immediately when the product was in the UHT heating system. Therefore, EN test 2 is not suitable as enteral tube feed for providing the target protein density to ICU patients who have to receive sufficiently sterile solutions.

EN test 1 however was stable against UHT treatment.

| **NUTRIENTS** | | | **EN TEST 1** | | **EN TEST 2** |
|---|---|---|---|---|---|
| **Values per** | | | **100 ml** | | **100 ml** |
| **Energy** | | kcal | 122 | | 122 |
| **Caloric density** | | kcal/ml | 1,2 | | 1,2 |
| **Water** | | ml | 80,5 | | 80,5 |
| **Osmolarity** | | mosmol/l | 600 | | 600 |
| **Protein** | | **33 Energy % g** | 10,0 | | 10,0 |
| | - thereof casein/whey | % | 100% whey protein hydrolysate | | 100% whey protein consentrate (non-hydrolysed) |
| **Fat** | | **24 Energy % g** | 3,2 | | 3,2 |
| thereof | | | | | |
| - MCT | | 9.4 Energy % g | 1,28 | | 1,28 |
| - LCT | | | 1,92 | | 1,92 |
| | - SFA¹⁾²⁾ | 2.8 Energy % g | 0,38 | | 0,38 |
| | - MUFA¹⁾ | | 6.0 Energy % g | 0,82 | 0,82 |
| | - PUFA¹⁾ | | 5.3 Energy % g | 0,72 | 0,72 |
| | | - Linoleic acid | 9 | 0,20 | 0,20 |
| | | - α-Linolenic acid | 9 | 0,09 | 0,09 |
| | | - EPA + DHA | 9 | 0,30 | 0,30 |
| | - n6/n3 Fatty Acids | | 9 | 0,5 | 0,5 |
| - Cholesterol | | | mg | ≤ 10 | ≤ 10 |
| **CHO (isomaltulose)** | | | **42 Energy % g** | 12,9 | 12,9 |
| **Dietary fibre** | | | **1 Energy % g** | 0,64 | 0,64 |
| | thereof | | | | |
| - tapioca dextrin fibre | | | 9 | 0,60 | 0,60 |
| - cellulose | | | 9 | 0,04 | 0,04 |
| - soluble/insoluble | | | % | 94 / 6 | 94 / 6 |
| - fermentable/nonferment able | | | % | 91/9 | 91/9 |
| **Protein** | | | **33 Energy % g** | 10,0 | 10,0 |
| | - thereof casein/whey | | % | 100% whey protein hydrolysate | 100% whey protein (non-hydrolysed) |

Clinical study protocol for the assessment of tolerability and clinical outcome of early protein intake when using the enteral tube feed herein (enteral tube feed according to the embodiments herein e.g. EN test 1) containing high protein and low fat content (per daily dose).

Patients: Inclusion criteria: Adult critically ill ventilated patients with indication for enteral nutrition and an expected ICU stay of one week or longer. Exclusion criteria: Contraindication for enteral nutrition (gut ischemia, obstruction or perforation distal from the nutritional tube); Expected intolerance for enteral nutrition (paralytic ileus); Short bowel syndrome; Child C liver cirrhosis or acute liver failure; Dialysis dependency; Requiring other specific enteral nutrition for medical reason; BMI > 35 kg/m2; Extensive treatment limitations.

Nr of patients: 20 patients receiving more than five full days of enteral nutrition with the study formula

Intervention: The study formula is started within 24-h after ICU admission, as soon as the circulation has been stabilized. Stable vasopressor support is no contraindication for the start of nutrition. Nutrition is primarily administered by the gastric tube at a rate of 20 ml/h and speed of administration is increased up to target if gastric retention is 250 ml or less. When gastric retention > 250 ml twice, erythromycin is added as a prokinetic. If gastric retention remains, a duodenal tube is inserted. Target protein intake: 1.2 g/kg preadmission body weight. Duration of the intervention: The study formula is administered for a maximum of 7 days, or less when the patient can eat normally, is discharged from the intensive or medium care unit to the normal ward or dies. Primary endpoint: Time to target protein intake compared to control. Secondary endpoint: Nr/% of patients with protein intake <1.0 or >1.4 g/kg/day

Good tolerance is inter alia reflected by easy digestion and absorption, good gastro intestinal tolerance. The time to reach the target protein intake of 1.0, 1.1 and/or (preferred target protein intake) 1.2 g/kg preadmission body weight is recorded. Reaching of higher protein intakes is recorded as appropriate.

**TABLE 2: Vitamins**

| | Minimun per 100kcal | Maximum per 100kcal |
|---|---|---|
| Vitamin A (µg RE) | 35 | 180 |
| Vitamin D (µg) | 0,5 | 3 |
| Vitamin K (µg) | 3,5 | 20 |
| Vitamin C (mg) | 2,2 | 22 |
| Thiamin (mg) | 0,06 | 0,5 |
| Riboflavin (mg) | 0,08 | 0,5 |
| Vitamin B6 (mg) | 0,08 | 0,5 |
| Niacin (mg EN) | 0,9 | 3 |
| Folic acid (µg) | 10 | 50 |
| Vitamin B12 (µg) | 0,07 | 0,7 |
| Pantothenic acid (mg) | 0,15 | 1,5 |
| Biotin (µg) | 0,75 | 7,5 |
| Vitamin E (mg α-TE) | 0,5 | 3 |

**TABLE 3: Minerals**

| | Minimum per 100kcal | Maximum per 100kcal |
|---|---|---|
| Sodium (mg) | 30 | 175 |
| Chloride (mg) | 30 | 175 |
| Potassium (mg) | 80 | 295 |
| Calcium (mg) | 35 | 250 |
| Phosphorus (mg) | 30 | 80 |
| Magnesium (mg) | 7,5 | 25 |
| Iron (mg) | 0,5 | 2,0 |
| Zinc (mg) | 0,5 | 1,5 |
| Copper (µg) | 60 | 500 |
| Iodine (µg) | 6,5 | 35 |
| Selenium (µg) | 2,5 | 10 |
| Manganese (mg) | 0,05 | 0,5 |
| Chromium (µg) | 1,25 | 15 |
| Molybdenum (µg) | 3,5 | 18 |
| Flouride (mg) | - | 0,2 |

## Claims

1. Enteral tube feed comprising a lipid component, a carbohydrate component and
at least 30 EN % of a protein component based on the total energy content of the enteral tube feed,
wherein the protein component comprises a peptide fraction consisting of di-, tri- and oligopeptides with a molecular weight of at most 1kD,
wherein the peptide fraction provides at least 10 EN% of the total energy content of the enteral composition,
wherein the carbohydrate component consists of at least 50wt% isomaltulose, wherein the enteral tube feed comprises 10-12 wt% protein based on the total weight of the enteral tube feed and
wherein the enteral tube feed has an energy density of 1.1-1.4 kcal/mL for use in prevention and treatment of protein malnutrition in ICU patients.

2. Enteral tube feed for use according to claim1, wherein the administration is started no later than 48 hours after admission to the ICU.

3. Enteral tube feed for use according to any of the preceding claims, wherein the use is further specified in providing a daily dose of calories of 20 - 25 kcal/kg BW/day in the acute phase and an increased daily dose of calories of 25 - 30 kcal/kg BW/day in the stabilized patient.

4. Enteral tube feed for use according to any of the preceding claims, wherein the use is further specified in providing a daily dose of protein of at least 1.0 g/kg BW/day at least in the acute phase.

5. Enteral tube feed comprising a lipid component, a carbohydrate component and at least 30 EN % of a protein component based on the total energy content of the enteral tube feed, wherein the protein component comprises a peptide fraction consisting of di-, tri- and oligopeptides with a molecular weight of at most 1kD, wherein the peptide fraction provides at least 10 EN% of the total energy content of the enteral tube feed , wherein the carbohydrate component consists of at least 50wt% isomaltulose, wherein the enteral tube feed comprises 10-12 wt% of protein based on the total weight of the enteral tube feed, and wherein the enteral tube feed has an energy density of 1.1-1.4 kcal/mL.

6. Enteral tube feed according to any of the preceding claims, wherein the lipid component provides at most 35 EN% based on the total energy content of the enteral tube feed.

7. Enteral tube feed according to any of the preceding claims wherein the lipid component comprises at least 30 wt% of MCT based on the total weight of the lipid component.

8. Enteral tube feed according to any of the preceding claims, wherein the lipid component comprises 30 - 50 wt% MCT oil, 20 - 40 wt% fish oil and 20 - 40 wt% vegetable oil.

9. Enteral tube feed according to any of the preceding claims, wherein the carbohydrate component comprises at least 50 wt% isomaltulose, 5 -20 wt% of starch and 20-40 wt% of further oligo- and polysaccharides.

10. Enteral tube feed according to any of the preceding claims wherein the lipid component provides 20 - 30 EN %, the carbohydrate component provides 30 - 50 EN % and the protein component provides 30 - 40 EN % each based on the total energy content of the enteral tube feed.

11. Enteral tube feed according to any of the preceding claims comprising 75-85 ml water / 100mL

12. The enteral tube feed of any of the preceding claims comprising a fibre mixture consisting of 80-99 wt% of soluble dietary fibre and 1-20 wt% of insoluble dietary fibre based on the total weight of dietary fibre.

13. The enteral tube feed of any of the preceding claims comprising a fibre mixture consisting of 80-99 wt% of fermentable dietary fibre and 1-20 wt% of non-fermentable dietary fibre based on the total weight of dietary fibre.

14. The enteral tube feed of any of the preceding claims having an osmolarity of 500 - 700 mosmol/L.

## Patentansprüche

1. Enterale Sondennahrung, die eine Lipidkomponente, eine Kohlenhydratkomponente und mindestens 30 EN % einer Proteinkomponente, bezogen auf den Gesamtenergiegehalt der enteralen Sondennahrung, umfasst,
wobei die Proteinkomponente eine Peptidfraktion umfasst, die aus Di-, Tri- und Oligopeptiden mit einem Molekulargewicht von höchstens 1 kD besteht,
wobei die Peptidfraktion mindestens 10 EN % des Gesamtenergiegehalts der enteralen Zusammensetzung bereitstellt,
wobei die Kohlenhydratkomponente zu mindestens 50 Gew.-% aus Isomaltulose besteht,
wobei die enterale Sondennahrung 10-12 Gew.-% Protein, bezogen auf das Gesamtgewicht der enteralen Sondennahrung, umfasst und
wobei die enterale Sondennahrung eine Energiedichte von 1,1-1,4 kcal/mL aufweist,
zur Verwendung bei der Vorbeugung und Behandlung von Proteinmangelernährung bei Patienten auf der Intensivstation.

2. Enterale Sondennahrung zur Verwendung nach Anspruch 1, wobei mit der Verabreichung spätestens 48 Stunden nach Aufnahme auf die Intensivstation begonnen wird.

3. Enterale Sondennahrung zur Verwendung nach einem der vorhergehenden Ansprüche, wobei die Verwendung ferner in der Bereitstellung einer täglichen Kaloriendosis von 20-25 kcal/kg Körpergewicht/Tag in der akuten Phase und einer erhöhten täglichen Kaloriendosis von 25-30 kcal/kg Körpergewicht/Tag beim stabilisierten Patienten besteht.

4. Enterale Sondennahrung zur Verwendung nach einem der vorhergehenden Ansprüche, wobei die Verwendung ferner in der Bereitstellung einer täglichen Proteindosis von mindestens 1,0 g/kg Körpergewicht/Tag zumindest in der akuten Phase besteht.

5. Enterale Sondennahrung, die eine Lipidkomponente, eine Kohlenhydratkomponente und mindestens 30 EN % einer Proteinkomponente, bezogen auf den Gesamtenergiegehalt der enteralen Sondennahrung, umfasst, wobei die Proteinkomponente eine Peptidfraktion umfasst, die aus Di-, Tri- und Oligopeptiden mit einem Molekulargewicht von höchstens 1 kD besteht, die Peptidfraktion mindestens 10 EN % des Gesamtenergiegehalts der enteralen Sondennahrung bereitstellt, die Kohlenhydratkomponente zu mindestens 50 Gew.-% aus Isomaltulose besteht, die enterale Sondennahrung 10-12 Gew.-% Protein, bezogen auf das Gesamtgewicht der enteralen Sondennahrung, umfasst und die enterale Sondennahrung eine Energiedichte von 1,1-1,4 kcal/mL aufweist.

6. Enterale Sondennahrung nach einem der vorhergehenden Ansprüche, wobei die Lipidkomponente höchstens 35 EN %, bezogen auf den Gesamtenergiegehalt der enteralen Sondennahrung, bereitstellt.

7. Enterale Sondennahrung nach einem der vorhergehenden Ansprüche, wobei die Lipidkomponente mindestens 30 Gew.-% MCT, bezogen auf das Gesamtgewicht der Lipidkomponente, umfasst.

8. Enterale Sondennahrung nach einem der vorhergehenden Ansprüche, wobei die Lipidkomponente 30-50 Gew.-% MCT-Öl, 20-40 Gew.-% Fischöl und 20-40 Gew.-% Pflanzenöl umfasst.

9. Enterale Sondennahrung nach einem der vorhergehenden Ansprüche, wobei die Kohlenhydratkomponente mindestens 50 Gew.-% Isomaltulose, 5-20 Gew.-% Stärke und 20-40 Gew.-% weitere Oligo- und Polysaccharide umfasst.

10. Enterale Sondennahrung nach einem der vorhergehenden Ansprüche, wobei die Lipidkomponente 20-30 EN %, die Kohlenhydratkomponente 30-50 EN % und die Proteinkomponente 30-40 EN %, jeweils bezogen auf den Gesamtenergiegehalt der enteralen Sondennahrung, bereitstellt.

11. Enterale Sondennahrung nach einem der vorhergehenden Ansprüche, die 75-85 mL Wasser/100 mL umfasst.

12. Enterale Sondennahrung nach einem der vorhergehenden Ansprüche, umfassend eine Ballaststoffmischung, die zu 80-99 Gew.-% aus löslichen Ballaststoffen und zu 1-20 Gew.-% aus unlöslichen Ballaststoffen, bezogen auf das Gesamtgewicht der Ballaststoffe, besteht.

13. Enterale Sondennahrung nach einem der vorhergehenden Ansprüche, umfassend eine Ballaststoffmischung die zu 80-99 Gew.-% aus fermentierbaren Ballaststoffen und zu 1-20 Gew.-% aus nicht fermentierbaren Ballaststoffen, bezogen auf das Gesamtgewicht der Ballaststoffe, besteht.

14. Enterale Sondennahrung nach einem der vorhergehenden Ansprüche, die eine Osmolarität von 500-700 mosmol/L aufweist.

## Revendications

1. Alimentation pour sonde entérale comprenant un composant lipidique, un composant glucidique et au moins 30 EN %. d'un composant protéique sur la base de la teneur énergétique totale de l'alimentation pour sonde entérale,
dans laquelle le composant protéique comprend une fraction peptidique constituée de di-, tri- et oligopeptides avec un poids moléculaire d'au plus 1 kD,
dans laquelle la fraction peptidique fournit au moins 10 EN %. de la teneur énergétique totale de la composition entérale,
dans laquelle le composant glucidique est constitué d'au moins 50 % en poids d'isomaltulose, dans laquelle l'alimentation pour sonde entérale comprend 10 à 12 EN % poids de protéines par rapport au poids total de l'alimentation pour sonde entérale et
dans laquelle l'alimentation pour sonde entérale a une densité énergétique de 1,1 à 1,4 kcal/mL
destinée à être utilisée dans la prévention et le traitement de la malnutrition protéique chez les patients des unités de soins intensifs.

2. Alimentation pour sonde entérale destinée à être utilisée selon la revendication 1, dans laquelle l'administration démarre au plus tard 48 heures après l'admission à l'unité de soins intensifs.

3. Alimentation pour sonde entérale destinée à être utilisée selon l'une quelconque des revendications précédentes, dans laquelle l'utilisation vise en outre à fournir une dose quotidienne de calories de 20 à 25 kcal/kg BW/jour dans la phase aiguë et d'une dose quotidienne accrue de calories de 25 à 30 kcal/kg BW/jour chez le patient stabilisé.

4. Alimentation pour sonde entérale destinée à être utilisée selon l'une quelconque des revendications précédentes, dans laquelle l'utilisation vise en outre à fournir une dose quotidienne de protéines d'au moins 1,0 g/kg BW/jour, au moins dans la phase aiguë.

5. Alimentation pour sonde entérale comprenant un composant lipidique, un composant glucidique et au moins 30 **EN** %. d'un composant protéique sur la base de la teneur énergétique totale de l'alimentation pour sonde entérale, dans laquelle le composant protéique comprend une fraction peptidique constituée de di-, tri- et oligopeptides avec un poids moléculaire d'au plus 1 kD, dans laquelle la fraction peptidique fournit au moins 10 **EN** %. de la teneur énergétique totale de l'alimentation pour sonde entérale, dans laquelle le composant glucidique est constitué d'au moins 50 % en poids d'isomaltulose, dans laquelle l'alimentation pour sonde entérale comprend 10 à 12 % en poids de protéines par rapport au poids total de l'alimentation pour sonde entérale, et dans laquelle l'alimentation pour sonde entérale a une densité énergétique de 1,1 à 1,4 kcal/mL.

6. Alimentation pour sonde entérale selon l'une quelconque des revendications précédentes, dans laquelle le composant lipidique fournit d'au plus 35 EN %. sur la base de la teneur énergétique totale de l'alimentation entérale.

7. Alimentation pour sonde entérale selon l'une quelconque des revendications précédentes dans laquelle le composant lipidique comprend au moins 30 % en poids de MCT par rapport au poids total du composant lipidique.

8. Alimentation pour sonde entérale selon l'une quelconque des revendications précédentes, dans laquelle le composant lipidique comprend 30 à 50 % en poids d'huile MCT, 20 à 40 % en poids d'huile de poisson et 20 à 40 % en poids d'huile végétale.

9. Alimentation pour sonde entérale selon l'une quelconque des revendications précédentes, dans laquelle le composant glucidique comprend au moins 50 % en poids d'isomaltulose, 5 à 20 % en poids d'amidon et 20 à 40 % en poids d'autres oligo- et polysaccharides.

10. Alimentation pour sonde entérale selon l'une quelconque des revendications précédentes dans laquelle le composant lipidique représente 20 à 30 EN %., le composant glucidique fournit 30 à 50 EN %. et le composant protéique 30 à 40 EN %. chacun sur la base de la teneur énergétique totale de l'alimentation pour sonde entérale.

11. Alimentation pour sonde entérale selon l'une quelconque des revendications précédentes, comprenant 75 à 85 mL d'eau/100 mL.

12. Alimentation pour sonde entérale selon l'une quelconque des revendications précédentes comprenant un mélange de fibres composé de 80 à 99 % en poids de fibres alimentaires solubles et de 1 à 20 % en poids de fibres alimentaires insolubles par rapport au poids total des fibres alimentaires.

13. Alimentation pour sonde entérale selon l'une quelconque des revendications précédentes comprenant un mélange de fibres composé de 80 à 99 % en poids de fibres alimentaires fermentables et de 1 à 20 % en poids de fibres alimentaires non fermentables par rapport au poids total des fibres alimentaires.

14. Alimentation pour sonde entérale selon l'une quelconque des revendications précédentes ayant une osmolarité de 500 à 700 mosmol/L.
